# EUROPEAN PATENT APPLICATION

(11) **EP 2 020 240 A1**
(43) Date of publication of application: **04.02.2009**
(21) Application number: 07113437.3
(22) Date of filing: 30.07.2007
(51) Int. Cl.: A61K 39/00, A61P 25/28, C12N 15/85, C07K 14/47

(54) **Methods and substances for the treatment of Alzheimer's**

(71) Applicant: Paul-Ehrlich-Institut Bundesamt für Sera und Impfstoffe, 63225 Langen (DE)
(72) Inventor: The designation of the inventor has not yet been filed
(74) Representative: Vossius & Partner

(57) **Abstract**

The present invention relates to a combination of one or more nucleic acids encoding, (a) in a first open reading frame (i) a promoter, (ii) an amyloid peptide, (iii) a cell surface targeting signal, (iv) a transmembrane anchoring domain and (b) in a second open reading frame (i) a group specific antigen (gag). Also the invention relates to a pharmaceutical composition and a vaccine comprising Aβ-retroparticles.

## Description

Alzheimer's disease (AD) is a neurodegenerative disorder which constitutes the major cause of dementia. It is estimated that 24 million people worldwide presently suffer from dementia with approximately 5 million in the European Union. The precise cause of neurodegeneration in AD is currently most intensely investigated. Based on current models, the beta-amyloid protein (Aβ), which is deposited as plaques in the brain of AD patients, is the causative agent. Presently, there is no curative treatment for AD. Medicinal products available, such as modulators of neurotransmitter systems, reduce the speed of disease progress without affecting the underlying pathogenetic process. In 1999, Schenk and co-workers described that Aβ plaques are reduced upon immunisation with the Aβ42 peptide in a transgenic AD mouse model. Moreover, reduction in plaque load markedly lowered the extent of AD-like neuropathology and improved learning in these mice. However, initial clinical trials which used aggregated Aβ1- 42 as antigen and adjuvant, had to be stopped in Phase II due to aseptic meningoencephalitis in 6% of the treated patients. Further investigations suggested that meningoencephalitis correlated with central nervous system (CNS) infiltrating T cells. As a consequence, more recent studies now aim for the induction of strong B cell responses without triggering T cell responses. Therapeutic vaccination is under development in many laboratories world-wide. None of the products have been authorised for marketing so far, nor gone into final phase clinical trials. The different vaccines cover a broad range of entities, including peptides mixed with different forms of adjuvant and genetic vaccines, such as plasmids or viral vectors, and N-terminal amino acid residues of Aβ42 displayed on phage particles (Novartis, Cytos), on scaffold proteins or adjuvant (Wyeth, Elan), or on papillomavirus particles. However, all these approaches require repeated dosing and thus are at risk to induce immunotoxic effects (acute shock syndrome, cytokine storm, encephalitis). Thus, only when clinical safety and efficacy data are available, it will be possible to decide which of the strategies results in an acceptable risk/benefit ratio to pursue development towards a marketable medicinal product.

Serious adverse effects consistent with meningoencephalitis resulted in the abrupt discontinuation of a recent clinical AD vaccination trail. Notably, AD vaccines tested so far were not very immunogenic and needed application together with adjuvants that are also used in animal models to induce experimental autoimmune encephalomyelitis. It was speculated that by avoiding T cell stimulation, serious adverse effects can be diminished. However, in absence of T help antibody responses are dominated by immunoglobulins of the M subclass (IgM), and thus are short-lived, of low binding affinity and not very effective in protection against AD.

Solving both problems the inventors have developed a vaccine that is very immunogenic in the absence of adjuvants and in which T helper cell determinants are provided in the context of the carrier retroparticle. Aβ-specific antibody responses are induced that show a long-lived, T helper dependent, IgG switch and Aβ-retroparticles allow therapeutic but also preventive vaccination. Such properties have not been described for other anti-AD vaccines, so far.

### DESCRIPTION OF THE INVENTION

The invention relates to a combination of one or more nucleic acids encoding, in a first open reading frame, (i) a promotor, (ii) an amyloid peptide, (iii) a cell surface targeting signal, (iv) a transmembrane anchoring domain and in a second open reading frame, (i) a group-specific antigen (gag) and optionally ii) the polymerase gene (pol).

### DEFINITIONS

Aβ1-15 peptide: an Aβ1-15 peptide as used herein refers to peptides having a sequence corresponding to the human Aβ1-15 sequence, or homologuous to the human Aβ1-15 sequence. Sequences homologuous to the Aβ1-15 sequence include, but are not limited to, the Aβ1-15 sequences of other species and hereby including, but are not limited to, the sequence of primate, rabbit, guinea pig, Xenopus Laevis, frog, mouse and rat. The Aβ1-15 sequences from Xenopus Laevis, frog, mouse and rat Aβ1. The Aβ1-15 sequences from Xenopus Laevis or frog, although differing from human Aβ1-15 at some positions are still considered to be homologuous to Aβ1-15 in accordance with this definition.

Adjuvant: The term "adjuvant" as used herein refers to non-specific stimulators of the immune response or substances that allow generation of a depot in the host which when combined with the vaccine and pharmaceutical composition, respectively, of the present invention may provide for an even more enhanced immune response. A variety of adjuvants can be used. Examples include complete and incomplete Freund's adjuvant, aluminum hydroxide and modified muramyldipeptide. Further adjuvants are mineral gels such as aluminum hydroxide, surface active substances such as lysolecithin, pluronic poplyols, polyanions, peptides, or oil emulsions, key hole llimpet hemocyanins, dinitrophenol and potentially useful human adjuvants such as BCG (bacille Calmette-Guerin) and *Croynebacterium parvum*. Such adjuvants are also well known in the art. Further adjuvants that can be administered with the compositions of the invention include, but are not limited to, monophosphoryl lipid immunomodulator, AdjuVax 100a, QS-21, QS-18, CRL1005, aluminum salts (Alum), MF-59, OM-174, OM-197, OM-294, and virosomal adjuvant technology. The adjuvants can also comprise a mixture of these substances.

Immunologically active saponin fractions having adjuvant activity derived from the bark of the South American tree Quillaja Saponaria Molina are known in the art. For example QS21, also known as QA21, is an Hplc purified fraction from the Quillaja Saponaria Molina tree and its method of its production is disclosed (as QA21) in U.S. Pat. No. 5,057,540. A preferred derivative is 3 de-o-acylated monophosphoryl lipid A, and is known from British Patent No. 2220211. Further preferred adjuvants are described in WO00/00462, the disclosure of which is herein incorporated by reference.

Antigen: As used herein, the term "antigen" refers to a molecule capable of being bound by an antibody or a T cell receptor (TCR) if presented by MHC molecules. The term "antigen", as used herein, also encompasses T-cell epitopes. An antigen is additionally capable of being recognized by the immune system and/or being capable of inducing a humoral immune response and/or cellular immune response leading to the activation of B- and/or T-lymphocytes. This may, however, require that, at least in certain cases, the antigen contains or is linked to a Th cell epitope and is given in adjuvant. An antigen can have one or more epitopes (B- and T-epitopes). The specific reaction referred to above is meant to indicate that the antigen will preferably react, typically in a highly selective manner, with its corresponding antibody or TCR and not with the multitude of other antibodies or TCRs which may be evoked by other antigens. Antigens as used herein may also be mixtures of several individual antigens.

Immune Response: As used herein, the term "immune response" refers to a humoral immune response and/or cellular immune response leading to the activation or proliferation of B- and/or T-lymphocytes and/or and antigen presenting cells. In some instances, however, the immune responses may be of low intensity and become detectable only when using at least one substance in accordance with the invention. "Immunogenic" refers to an agent used to stimulate the immune system of a living organism, so that one or more functions of the immune system are increased and directed towards the immunogenic agent. An "immunogenic polypeptide" is a polypeptide that elicits a cellular and/or humoral immune response, whether alone or linked to a carrier in the presence or absence of an adjuvant. Preferably, antigen presenting cell may be activated.

A substance which "enhances" an immune response refers to a substance in which an immune response is observed that is greater or intensified or deviated in any way with the addition of the substance when compared to the same immune response measured without the addition of the substance.

Immunization: As used herein, the term "immunize" or "immunization" or related terms refer to conferring the ability to mount a substantial immune response (comprising antibodies and/or cellular immunity such as effector CTL) against a target antigen or epitope. These terms do not require that complete immunity be created, but rather that an immune response be produced which is substantially greater than baseline. For example, a mammal may be considered to be immunized against a target antigen if the cellular and/or humoral immune response to the target antigen occurs following the application of methods of the invention.

Polypeptide: As used herein, the term "polypeptide" refers to a molecule composed of monomers (amino acids) linearly linked by amide bonds (also known as peptide bonds). It indicates a molecular chain of amino acids and does not refer to a specific length of the product.

Vaccine: As used herein, the term "vaccine" refers to a formulation which contains the composition of the present invention and which is in a form that is capable of being administered to an animal. Typically, the vaccine comprises a conventional saline or buffered aqueous solution medium in which the composition of the present invention is suspended or dissolved. In this form, the composition of the present invention can be used conveniently to prevent, ameliorate, or otherwise treat a condition. Upon introduction into a host, the vaccine is able to provoke an immune response including, but not limited to, the production of antibodies and/or cytokines and/or the activation of cytotoxic T cells, antigen presenting cells, helper T cells, dendritic cells and/or other cellular responses. However, as outlined below the advantage of the present invention is the reduction of the T-cell response.

Virus-like particle (VLP): As used herein, the term "virus-like particle" refers to a structure resembling a virus particle. Moreover, a virus-like particle in accordance with the invention is non replicative and non-infectious since it lacks all or part of the viral genome, in particular the replicative and infectious components of the viral genome. A virus-like particle in accordance with the invention contains no nucleic acids from the virus genome.

A "cell surface targeting signal" also called signal peptide, as used herein refers to a short 3-60 amino acids long peptide chain that directs the post-translational transport of proteins which are synthesised in the cytosol to the endoplasmic reticulum from where they are transported to the cell surface membrane. It exists at the amino terminus of a protein. The protein is guided to the ER by a signal-recognition particle, which binds to the signal peptide. The signal peptide is cleaved from the protein by signal peptidase after the protein has been translocated to the ER.

A "transmembrane anchoring domain" as used herein refers to a transmembrane alpha helix of a transmembrane protein. A transmembrane anchoring domain is usually about 20 amino acids in length, but can be longer or shorter. A transmembrane anchoring domain is any three-dimensional protein structure which is thermodynamically stable in membrane. This may be a single alpha helix, a stable complex of several transmembrane alpha helices, a transmembrane beta barrel, a beta-helix of gramicidin A, or any other structure.

A "group specific antigen" as used herein refers to any polypeptide that is being encoded by the gag gene of the members of the family *Retroviridae*. This polypeptide covers the core structural proteins of a retrovirus.

In a preferred embodiment said first open reading frame and said second open reading frame are encoded on two different vectors.

The amyloid peptide has a length between 42 and 10 amino acids. The amyloid peptide may have 42 amino acids, 38 amino acids, 32 amino acids, 24 amino acids, 18 amino acids, 15 amino acids, 10 amino acids or 5 amino acids. In principle, it may be from the central region of the full-length peptide or from the C- or N-terminal region. The N-terminal region is preferred.

In a preferred embodiment said amyloid peptide is selected from 42 amino acid peptide, a 15 amino acid fragment from the N-terminal region, a 10 amino acid fragment from the N-terminal region or any shorter peptide of at least the N-terminal five amino acids of the amyloid peptide.

In one embodiment of said combination according to the invention, (a) said first open reading frame additionally comprises, (i) one or more immunological tags for detection and (b) said one or more nucleic acids additionally comprises, (i) a selectable marker gene for expression in mammalian cells.

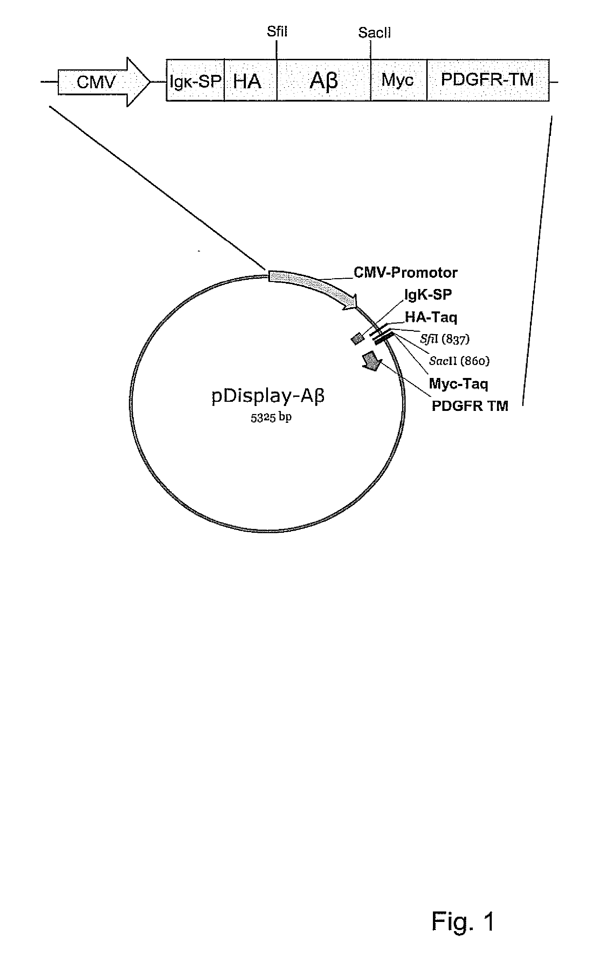

"Myc" (see Fig. 1) which is located between the Aβ-peptide and the transmembrane anchoring domain (PDGFR-TM) may serve as a linker. However, other peptides may also serve as liner. Per sé a linker is optional. One preferred linker is the so-called glycin-serin linker (G₄S-linker). Further, the Pol-gene may be present on the same vector as the GAG gene.

In a preferred embodiment (a) said promoter is selected from the group of, human cytomegalovirus (CMV) immediate-early promoter/enhancer, the simian virus 40 (SV40) promoter/enhancer, retroviral long terminal repeat (LTR) promoter/enhancer, or any other mammalian promoter/enhancer, (b) said cell surface targeting signal is selected from the group of, murine Ig κ-chain leader sequence, a mammalian immunoglobuline leader sequence, a leader sequence of retroviral envelope proteins, a leader sequence of other viral envelope proteins, and a leader sequence of any mammalian type I transmembrane protein or secreted protein, (c) said transmembrane anchoring domain is selected from the group of, platelet-derived growth factor receptor transmembrane domain (PDGFR-TM), a transmembrane anchoring domain of retroviral envelope proteins, a transmembrane anchoring domain of other viral envelope proteins, and of any mammalian type I transmembrane protein, (d) said group-specific antigen (gag) is selected from the group of murine leukemia virus (MLV) gag, the group of γ-retroviral gag proteins, the group of the human immunodeficiency I virus (HIV-I), the group of lentiviral gag proteins, and any gag protein of the family Retroviridae, and (e) said immunological tag is selected from the group of, hemagglutinin A epitope tag, myc epitope, or FLAG epitope.

In a preferred embodiment the Aβ-display protein is according to SEQ ID NO. 1, the Ig-kappa light chain signal peptide is according to SEQ ID NO. 2, the HA-Tag is according to SEQ ID NO. 3, the Aβ1-15 peptide is according to SEQ ID NO. 4, the c-myc-tag is according to SEQ ID NO. 5 and the transmembrane domain is according to SEQ ID NO. 6.

The invention relates to Aβ retroparticles selected from the group of Aβ retroparticles comprising an amyloiod peptide according to SEQ ID NO. 4 and additionally having a sequence according to SEQ ID NO: 1, 2, 3, 5, and 6 and Aβ retroparticles having a sequence which has 80%, 85%, 95% or preferably 98% sequence identity with said sequences.

In a further embodiment the invention relates to a kit comprising a combination according to the invention.

The invention relates to a method for producing a medicament or a vaccine comprising the steps of, (a) transfecting a mammalian cell line with the combination of the one or more nucleic acids according to the invention, (b) incubating the mammalian cells, (c) harvesting the supernatant and (d) isolating the Aβ-retroparticles from the supernatant, optionally the particles are quantified.

In a preferred embodiment the medicament or vaccine is directed against Alzheimer's disease.

In one embodiment the invention concerns a pharmaceutical composition comprising Aβ-retroparticles produced according to the method of the invention.

In a preferred embodiment the pharmaceutical composition is for the treatment of diseases associated with Aβ-aggregation. In a preferred embodiment the disease is selected from the group of Alzheimer's disease inclusion body myositis, amyotrophic lateral sclerosis, cerebral amyloid angiopathy.

The invention relates to a pharmaceutical composition comprising, Aβ-retroparticles comprising the following proteins or peptides, (i) an amyloid peptide, (ii) cell surface targeting signal (iii) a transmembrane anchoring domain, and (iv) a group-specific antigen (gag).

The amyloid peptide has a length between 42 and 5 amino acids. The amyloid peptide may have 42 amino acids, 38 amino acids, 32 amino acids, 24 amino acids, 18 amino acids, 15 amino acids, 10 amino acids or 5 amino acids. In principle, it may be from the central region of the full-length peptide or from the C- or N-terminal region. The N-terminal region is preferred.

It is preferred that the amyloid peptide is a 42 amino acid peptide, a 15 amino acid fragment from the N-terminal region, a 10 amino acid fragment from the N-terminal region.

It is most preferred that the amyloid peptide has an amino sequence selected from the group of SEQ ID NO.4.

In one embodiment the Aβ-retroparticles additionally comprise, one or more immunological tags for detection.

In a preferred embodiment of the pharmaceutical composition according to the invention, wherein (a) said promoter is selected from the group of, human cytomegalovirus (CMV) immediate-early promoter/enhancer, the simian virus 40 (SV40) promoter/enhancer, retroviral long terminal repeat (LTR) promoter/enhancer, or any other mammalian promoter/enhancer, (b) said cell surface targeting signal is selected from the group of, murine Ig κ-chain leader sequence, a mammalian immunoglobuline leader sequence, a leader sequence of retroviral envelope proteins, a leader sequence of other viral envelope proteins, and a leader sequence of any mammalian type I transmembrane protein or secreted protein, (c) said transmembrane anchoring domain is selected from the group of, platelet-derived growth factor receptor transmembrane domain (PDGFR-TM), a transmembrane anchoring domain of retroviral envelope proteins, a transmembrane anchoring domain of other viral envelope proteins, and of any mammalian type I transmembrane protein, (d) said group-specific antigen (gag) is selected from the group of murine leukemia virus (MLV) gag, the group of γ-retroviral gag proteins, the group of the human immunodeficiency I virus (HIV-I), the group of lentiviral gag proteins, or from and any gag protein of the family Retroviridae, and (e) said immunological tag is selected from the group of, hemagglutinin A epitope tag, myc epitope, or FLAG epitope.

It is preferred that the composition is devoid of nucleic acids from the original virus completely devoid of nucleic acids and/or free of a viral envelope protein.

The pharmaceutical composition according to the invention may comprise an agent selected from the group of, (a) an adjuvant, and (b) an acceptable pharmaceutical carrier Aβ-retroparticles may also be administered in a prime/boost.

The invention also relates to methods of treatment.

### FIGURE CAPTIONS

### Figure 1: Schematic representation of the expression plasmid encoding the Aβ-peptide

The pDisplay-Aβ expression plasmid derived from the pDisplay plasmid (Invitrogen) provides a CMV promoter, the Igκ signal peptide (Igκ-SP), the HA immunological tag, the Aβ1-15 peptide, the Myc immunological tag and the transmembrane domain of the PDGF receptor (PDGFR TM).

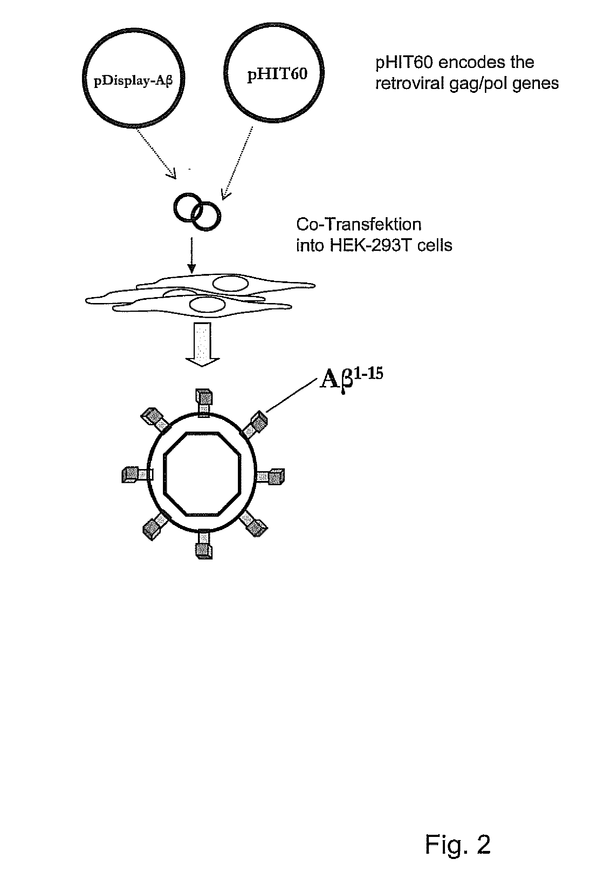

### Figure 2: Generation of Aβ1-15 retroparticles

HEK-293T cells are cotransfected with the plasmids pDisplay-Aβ and pHIT60 (encodes for retroviral gag and pol genes). The transfected cells release Aβ1-15 retroparticles which can be harvested from the cell supernatant. The particles are derived from murine leukaemia virus, containing a core structure composed of gag proteins and an envelope membrane. The Aβ1-15 peptide fused to the PDGFR transmembrane domain is inserted in the membrane.

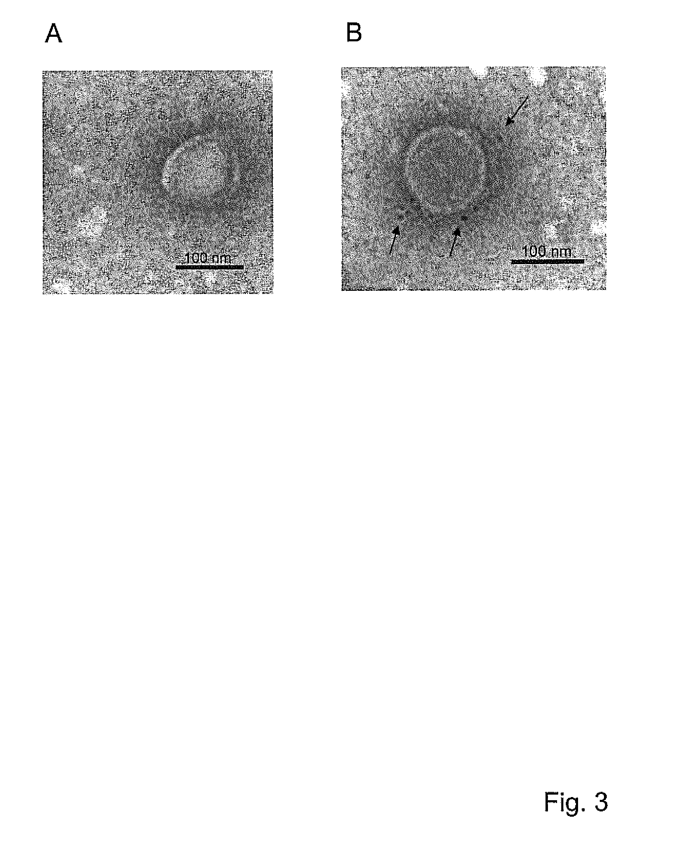

### Figure 3: Immunoelectron microscopic analysis of retroparticles

Retroparticles displaying the epidermal growth factor (EGF) (A) or Aβ1-15-retroparticles (B) were applied to glow-discharged carbon coated Formvar grids. Aβ was labeled by an anti-Aβ antibody and a gold-particle labeled secondary antibody (arrowheads). Arrows indicate gold particles bound to the Aβ1- 15 peptide displayed on the particle surface.

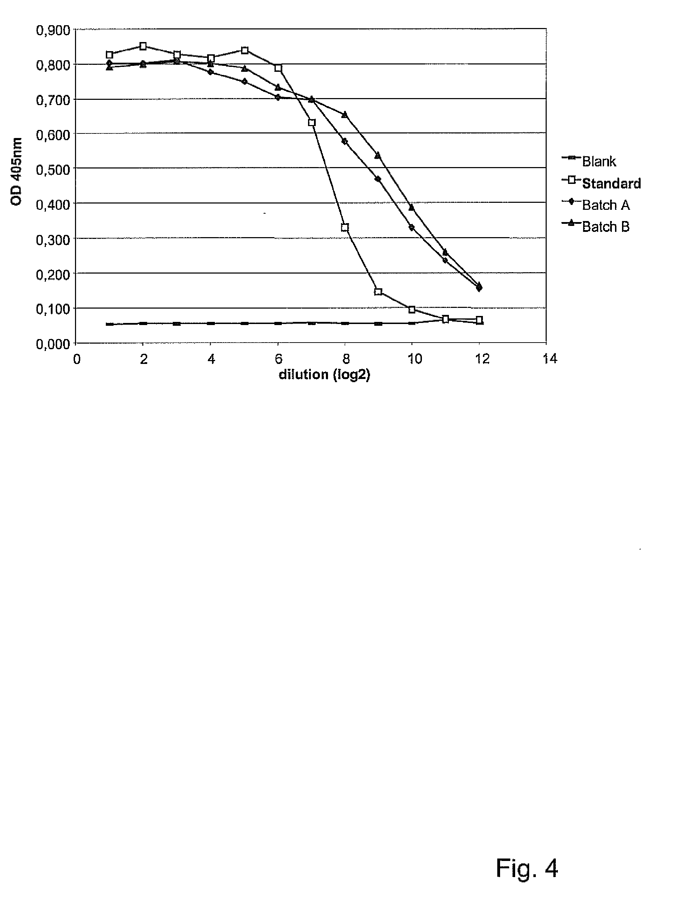

### Figure 4: Analysis of Aβ-retroparticles by ELISA

Two concentrated stocks of Aβ-retroparticles (filled triangle, filled diamond) were coated to ELISA plates at the indicated dilutions. As controls, plates were left empty (filled rectangle) or were coated with defined amounts of Aβ-peptide (open square). All samples were incubated with an anti-Aβ monoclonal antibody. The data demonstrate the reproducibility of the Aβ-retroparticle production.

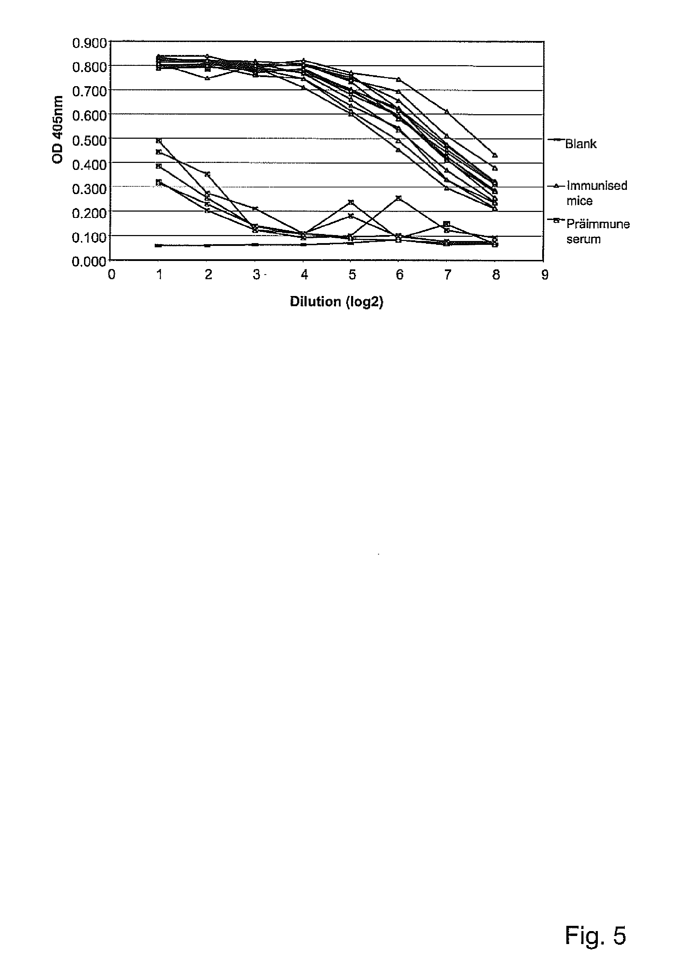

### Figure 5: Aβ-specifc antibody response in Aβ-retroparticle vaccinated mice

Sera from Aβ-retroparticle immunized APP23 mice were taken 10 days after the first boost immunization (open triangles). Samples were tested in log2 serial dilution (20-fold predilution) for the presence of Aβ-specific total antibodies. Titers ranged between 1:800 and 1:3000. Preimmune sera (crossed symbols) or Aβ-standard (filled sqares) were used as controls.

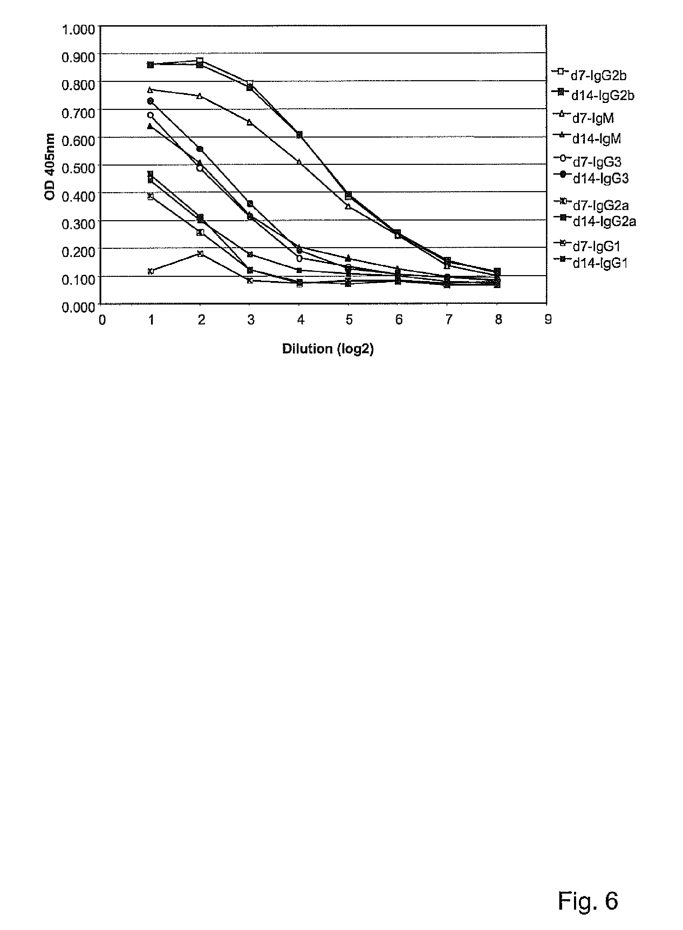

### Figure 6: Isotype profiles of Aβ-specific antibody responses in Aβ-retroparticle vaccinated mice

Sera from Aβ-retroparticle vaccinated mice were collected 7 (d7, open symbols) or 14 days (d14, filled symbols) after immunization. Serum samples were tested in log2 serial dilutions (20-fold predilution) for the presence of Aβ-specific IgM and indicated IgG subclass antibodies by ELISA.

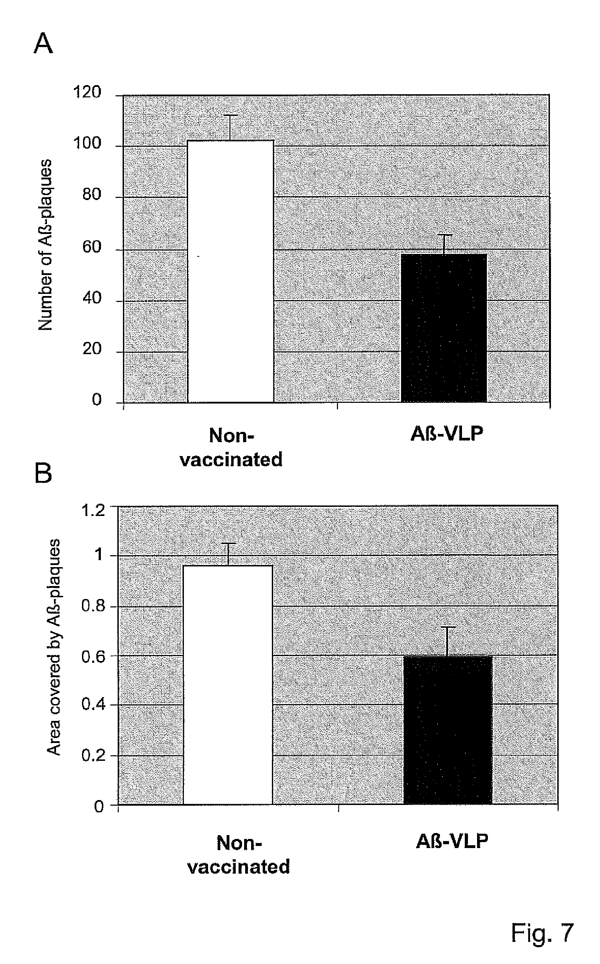

### Figure 7: Significant reduction of the plaque load in Alzheimer's disease model mice

APP23 mice were monthly immunised over a period of 6 months starting at 4 months of age. For immunization 10⁹ Aβ-retroparticles were injected into the tail vein in the absence of any adjuvant. At an age of 12 months mice were sacrificed and the amount of Aβ was quantified in brain sections by either staining the number of Aβ-plaques (A) or by quantifying the area covered by Aβ-plaques (B). Statistically significant reductions in the immunized group were observed (p < 0.05; Student's t-test).

To translate this approach into a vaccination strategy against AD, the coding sequence for Aβ1-15 was fused to the transmembrane domain of the platelet derived growth factor receptor (PDGFR). When incorporated into particles, the displayed Aβ became readily detectable by monoclonal antibodies (Fig. 3). The particles can be concentrated and quantified by ELISA revealing that 10⁹ particles display about 100 ng of Aβ (Fig.4). High levels of Aβ-specific antibodies were detected in AD model mice APP23 that had been immunised monthly over a period of 6 months, starting at the age of 4 months (Fig. 5). Remarkably, a strong immune response was observed already after a single injection in the absence of any adjuvant. Moreover, a statistically significant reduction of about 50% in the number of plaques per brain section and in the average area covered by Aβ42 plaque deposition was observed (Fig. 7). Thus, Aβ-retroparticles are able to induce an Aβ-specific immune response that prevents plaque deposition.

### EXAMPLES

### Example 1:

### Preparation of Aβ-retroparticles

Aβ-retroparticles are produced by co-transfection of HEK-293T cells with the plasmid pHIT60 encoding the MLV gag and pol genes, and the plasmid pDisplay-Aβ. The plasmid pDisplay-Aβ has been cloned by assembling two oligonucleotides SEQ ID NO. 7 SfiI-Aβ¹⁻¹⁵+ 5'-GATCGGCCCAGCCGGCCGATGCAGAATTCCG and SEQ ID NO. 8 SacII-Aβ¹⁻¹⁵- 5'-GACTCCGCGGCCGGCCCTCGATCGCTATGACAACACCGCCC, filling them with Klenow-Polymerase and cleaving with SfiI and SacII. The resulting DNA fragment coding for the N-terminal 15 amino acids of human Aβ was inserted into the restriction sites SfiI and SacII of pDisplay. The sequence of the resulting plasmid was verified by sequencing. 24 h before transfection 6.5x10⁶ HEK-293T cells (cultivated in DMEM with 4.5 g/l glucose, 3.7 g/l NaHCO3, 10% FCS, 1% glutamin, 0.5% penicillin/strepromycin) are seeded into a T75 flask. Transfection is performed with Lipofectamin 2000 according to the manufacturer's protocol (Invitrogen) using 9 µg of the plasmid pHIT60 and 9 µg of the plasmid pDisplay-Aβ. 48h after transfection 12 ml cell supernatant containing the Aβ-retroparticles is filtrated (0.45 µm filter) and centrifuged at 3600 rpm (Heraeus multifuge 3S-R) at 4°C for at least 16 h for concentration. The pellet is then resuspended in PBS Dulbecco/1% FCS (50µl/ T75-Flasche). For titration of the particle stocks, ELISA plates are coated with Aβ-retroparticles and the amount of the Aβ peptide displayed by the particles is quantified using an Aβ-specific monoclonal antibody. The number of particles is quantified by reverse transcriptase (RT) assay.

### Example 2:

### Immunoelectron microscopic analysis of retoparticles

For immunonegative staining, 20 µl of virus suspension was adsorbed to glow discharged carbon coated formvar grids for 2 minutes. After rinsing in PBS, grids were incubated with 2% BSA for 30 minutes and with the primary Aβ-specific monoclonal antibody 6E10 (Signet Labs) at a 1:100 dilution for 1h, respectively. After washing, grids were incubated with a 10nm gold labelled antibody for 30 minutes. Finally, immunolabeled viruses were negatively stained with 2% uranylacetate or phosphotungstate for 10 seconds. EM preparations were examined in a Zeiss EM 109 or 902 electron microscope and micrographs were taken on Kodak Estar electron microscope film.

### Example 3:

### Immunization of mice with Aβ-retroparticles

APP23 mice, 4 months of age, were immunized by intravenous injection of approximately 10¹¹ Aβ-retroparticles in 200 µl PBS. Booster injections were performed 14 days after primary immunization. Blood was taken at different time points to monitor the antibody reactivity.

### Example 4:

### Analysis of Aβ-specific antibodies by ELISA

ELISA plates were coated with 415 ng per well of human Aβ (Bachem) in PBS and blocked with 5% bovine serum albumin (BSA). Serial 2 fold serum dilutions in PBS, 0.1% Tween, 1% BSA were added. After 2 h incubation at room temperature, plates were thoroughly washed and 1:1000 diluted horse radish peroxidase (HRP)-conjugated polyclonal rabbit antibody directed against mouse Ig of the M subclass (Southern Biotec at a 1:500 dilution) or the IgG1 (Southern Biotec at a 1:500 dilution), IgG2a (Zymed, at a 1:1000 dilution) or IgG3 (Southern Biotec, at a 1:500 dilution) isotypes added. After 1 h incubation at room temperature plates were washed and for the detection of bound HRP-coupled antibodies substrate was added (0.5 mg/ml 2.2'-azino-di-ethyl-benzothiazolinsulfonate (Roche) in 0.1 M NaH₂PO₄, pH 4 and 30% H₂O₂). The optical density was determined at a wave length of λ= 405 nm.

### Example 5:

### Aβ-plaque quantification in brain slices of Aβ-retroparticle immunized mice

Determination of the Aβ-plaque burden is performed on fixed, paraffin embedded parasagital brain sections stained with an antibody specific for human Aβ (6E10, or NT12) as described (Sturchler-Pierrat, 1997). In detail, brain material is fixated by immersion with 4% formaldehyde followed by paraffin embedding and sectioning with a microtome. One complete brain half is taken for histological investigation. It is fixated in 4% PBS buffered formaldehyde solution and further processed for paraffin sectioning. The second half is usually prepared for biochemical investigation. The half is either frozen on dry ice, or brain areas of interest are manual dissected under stereomicroscopic control, weighted and frozen in small plastic tubes.

After dehydration of the fixed brain one or both hemispheres are embedded in paraffin. 40 µm sagittal sections are prepared and mounted onto glass slides. For immunohistochemical staining, sections are washed in PBS and then heated at 90°C in 0.1 M citric acid buffer for 15 to max 30 min by microwave treatment to enhance antigenicity. After washing in PBS, sections are blocked in goat serum for 20 min and then incubated in 80 ul primary antibody solution (1:2000 dilution) over night at 4°C. After washing in 0.01 M TBS-buffer, a 1:200 dilution of the secondary biotinylated antibody is added for 30 min. Finally, the sections are incubate in ABC reagent (*Vector Elite Kit)* and then developed in enzyme substrate (Vector Laboratories Kat No: SK-4200 for about 30 sec to maximal 3 min. Quantitative analysis is performed in a blinded fashion using a computer assisted system. To monitor potential inflammatory responses staining for infiltrating T cells (anti-CD5), astrogliosis (anti-GFAP), microgliosis (anti- CD11b, tomato lectin) and complement C3 is performed.

### Example 6:

### Determination of soluble and insoluble Aβ-load in brain tissue of Aβ-retroparticle immunised mice (ELISA)

Mouse brain hemispheres are weighted and homogenized in 100µl /mg brain homogenizing buffer (250mM Sucrose, 20mM Tris/HCl pH 7.4, 1mM EGTA+ Complete (Roche, Switzerland) ) using a Sartorius Potter S at 400rpm 20times up and down. 100µl each of the raw homogenate is diluted 1:10 in homogenizing buffer for further extraction. For extraction of soluble Aβ 1ml diluted homogenate is mixed with 1ml DEA-saline (Diethylamin 0.4% v/v, 100mM NaCl) and homogenized using a Sartorius Potter S at 1500rpm, 20 times up and down. To extract aggregated Aβ 1ml of diluted homogenate is mixed with 2.2ml formic acid and sonified using a Branson sonfier S-450A (Branson Inc., U.S.A.) level 4 output 40% for 1min. Both extractions are ultra-centrifuged at 4°C for 1h at 100,000x g using a Beckman ultra centrifuge (Beckman, U.S.A.). DEA extracts are buffered adding 1/10 0.5M Tris/HCl pH 6.8. Formic acid extracts are neutralized adding 4ml of 1M Tris/HCl, 0,5M Na₂HPO₄. Extracts were frozen in liq. N₂ and stored at -80°C.

Each extract is measured in triplicate using a highly specific sandwich-ELISA (TGC, Switzerland). Briefly, NUNC Maxisorb microtiter plates (Nunc, Denmark) are coated with capture antibodies in a concentration of 5µg/ml for 2h at 37°C, washed 3 times with washing buffer, blocked using StabilCoat® (Surmodics, U.S.A.), washed 3 times again. Sample-detection reaction is incubated over night at 4°C employing a biotinylated detection antibody for Aβ40 and for Aβ42 separately. Standardization is performed with standard dilution series of synthetic Aβ (Bachem, Switzerland) in a range of 125pg/ml up to 2000pg/ml to calculate concentrations.

50µl of each homogenate is used for each determination. After a 4-fold wash step detection is performed using a poly-Streptavidine-Horsereddish-Peroxidase conjugate (GE Healthcare, U.S.A.) and substrate reaction after a 5-fold wash step is performed using TMB (Pierce, U.S.A.) as a substrate and 1M H₂SO₄ as a stopping reagent.

Adsorption measurements are performed with a BioTek FLx800™ Multi-Detection Microplate Reader (BioTek, Germany) at 450nm. Antibodies used in this assay are directed against Aβ N-terminus (detection antibody) and against the C-termini of Aβ40 andAβ42 respictively (capture antibodies).

### Example 7:

### Aβ-plaqne deposit staining in post mortem brain sections of AD patients with sera from Aβ-retroparticle immunized mice

To assess whether antibodies produced in response to Aβ-retroparticles can recognize Aβ-amyloid deposited in brain tissue sections from APP23 mice and sections from human Alzheimer's disease brain will be stained with the antiserum. Essentially the same protocol as described in Example 5 will be followed.

### Example 8:

### Scale up of particle production and purification

Different possibilities will be followed to optimise the production process of Aβ-retroparticles.

### Generation of a stable producer cell line for Aβ-retroparticles

HT1080 and HEK-293 cells are transfected with plasmids pHIT60 encoding the MLV gag/pol genes and with plasmid pDAβ1-15 encoding the Aβ-display construct. After transfection cells are selected by neomycin and blasticidin to achieve stable genomic integration of both plasmids. About 100 single cell clones are grown up and tested for Aβ-retroparticle release. Clones allowing maximal particle production as assessed by RT-assay and Aβ-ELISA are selected for the production process.

### Production of Aβ-retroparticles from insect cells

Baculovirus and insect cells based expression of the Aβ-retroparticle structural proteins is an alternative approach to the particle generation in human cell lines, which might be advantageous with respect to virus safety issues and the possibility to produce in a non-tumorigenic cell line. First, recombinant baculoviruses for the MLV gag gene and for the pDAβ1-15 construct is generated. These are used for coinfection of SF-9 cells. From day 2 on, supernatant from the cells is harvested every 24 hours and the number of particles and the amount of Aβ antigen is quantified by ELISA. Western blot analysis and electron microscopy is performed to exclude any unwanted modifications of the particles. Immunogenicity of the particles is determined by vaccination of APP23 mice and by determining the Aβ-specific antibody titer in the serum of the animals one and two weeks after particle injection.

### Purification of Aβ-retroparticles

Separation of the virus-like particles from cellular membranes and cell debris is possible due to the higher density of virus-like particles that contain matrix protein and the capsid. Sucrose density gradients are run to separate virus-like particles and membranes. Fractions collected are analyzed by RT-assay and by Western blot against Aβ to identify the membrane and the virus-like particle containing fractions. These are then dialyzed against PBS and the amounts of Aβ present are quantified by ELISA.

### Scale-up of the Aβ-retroparticle production process

Once the basic conditions (production cell, concentration and purification) of the production process are established, a scale-up from single cell culture flasks to dish staples is performed. This reflects a scale up from about 5x10⁷ cells to about 5x10⁹ cells producing Aβ-retroparticles. A single Aβ-retroparticle batch comprises about 10¹¹ particles and about 10µg of displayed Aβ. This large scale Aβ-retroparticle preparation is then compared to the low-scale preparation for their immunogenicity in APP23 mice as described above.

### Example 9:

### Generation of Aβ1-7-retroparticles

Starting from the plasmid pD-Aβ1-15 overlap extension PCR is performed to delete the codons for the Aβ-residues 8-15 resulting in the plasmid pD-Aβ1-7. This plasmid is then used together with pHIT60 to produce Aβ1-7-retroparticles upon cotransfection of HEK-293T cells. Particles harvested are quantified by RT-assay and Aβ-specific ELISA. Equivalent amounts of Aβ1-7-retroparticles and Aβ1-15-retroparticles are used for the immunization of APP24 mice. Serum from the mice is removed about 10 days after injection and Aβ-specific antibodies are quantified by ELISA. A dose response curve is generated to quantify the immunogenicity of both types of Aβ-retroparticles. Moreover, the T cell response is determined from isolated splenocytes of the immunized mice by ELISPOT assay.

### Example 10:

### Determining the optimal application regimen

To determine the optimal route for immunisation, wild type C57/BL6 mice are immunized in triplicate with Aβ-retroparticles (10⁹ particles/100 ng Aβ) via the intravenous, intranasal, intramuscular and subcutanous route, which is followed by two consecutive rounds of boosting 2 and 4 weeks later. Aβ-specific serum titers are determined by ELISA coated with synthetic Aβ40 at 10, 24 and 38 days after the first injection of antigen. Cytokine profiles indicative of a proinflammatory Th1 (IL-2, INFγ), versus Th2 (IL-4, IL-5) immune responses are determined *ex vivo* and upon *in vitro* restimulation of T cells isolated from spleen cells of immunized mice.

Two types of regimens, e. g. prophylactic and therapeutic are studied to examine the effect of Aβ-retroparticle immunization. In addition, it will be determined whether a single dose vaccination is sufficient to induce high Aβ-specific antibody titers, remaining stably elevated until the experiment is terminated. For prophylactic intervention, immunization of 2 groups of mice at 3-4 months of age before the onset of plaque deposition is started. Group A receives a single dose vaccination, whereas group B receives in addition monthly boosts of Aβ-retroparticles until termination of the study at 12 months of age. For therapeutic intervention, we start immunization at 12 months of age (6 months after beginning of plaque deposition) with termination of the study at 18 months. Again, mice either receive a single dose vaccination (group C) or boosted monthly until termination of the study (group D). Throughout the whole study (groups A-D) transgenic mice, receiving EGF-displaying retroparticles are treated in parallel and serve as controls. Aβ-specific antibody titers are determined by ELISA from preimmune serum and 10 days after each immunization or boost, respectively. Animals receiving only a single dose vaccination are bled at similar time points to monitor a possible decline of Aβ-specific antibody titers.

### Example 11:

### Defining toxicity of AD vaccination in mice and non-human primates

Mice and non-human primates (Macaca mulata) are subjected to a repeated dose escalation study. Animals are treated with a high dose and two even 10-fold higher dosages of Aβ-retroparticles. Each dose is applied on four consecutive weeks. To study the impact of T helper cell triggering, retroparticles displaying the envelope protein G of Vesicular stomatitis virus (VSV) are used for boosting of Aβ hyperimmune mice. Under such conditions, Aβ-specific B cells are not triggered, whereas retroparticle-specific T helper cells are activated. Furthermore, Aβ hyperimmune mice are subjected to VSV infection. VSV is a very potent immunogen that cases T cell infiltration of the olfactory bulb and leads to massive B cell activation.
In addition, humanized mice are subjected to a repeated dose escalation study. Immunological parameters such as human cytokine profiles and blood cell counts are determined. Furthermore, an immune histological analysis is performed of secondary lymphoid organs and infiltration of the central nervous system is studied. It is studied whether upon Aβ-retroparticle immunization, T cell infiltration of the central nervous system can be observed in absence of B cells and whether in absence of either B or T cells toxicity signals are observed.

### Example 12:

### Behavioural and cognitive analysis of Aβ-retroparticle immunised mice

One month before sacrificing mice for Aβ load determination mice are behaviorally tested to assess whether Aβ-retroparticle immunization improves their cognitive performance. To this end, hippocampus-dependent spatial navigation is assessed in the Morris water-maze. Testing includes an acquisition phase, as well as a reversal phase with the platform moved to the opposite quadrant, as a read out for memory formation. Performance towards a visible platform (cued version of the test) is monitored, to assess any potential motor defects. Besides an age-dependent decline of cognitive functions, the APP23 model was previously shown to exhibit cage activity disturbances, reminiscent of circadian rhythm disturbances in Alzheimer patients. Therefore, it is also studied whether immunization ameliorates altered circadian locomotor activity. Home cage activity is recorded in individual cages equipped with IR-sensors. The sensors detect any locomotion and remain silent only when the mice are sleeping or grooming.

## Claims

1. Combination of one or more nucleic acids encoding,
a. in a first open reading frame,
i. a promotor
ii. an amyloid peptide
iii. a cell surface targeting signal
iv. a transmembrane anchoring domain
b. in a second open reading frame,
i. a group-specific antigen (gag).

2. Combination according to claim 1, wherein
said first open reading frame and said second open reading frame are encoded on two different vectors.

3. Combination according to claim 1 or 2, wherein said amyloid peptid is between 42 amino acids and 5 amino acids long.

4. Combination according to claims 1 to 3, wherein
said amyloid peptide has an amino acid sequence selected from the group of SEQ ID NO.4.

5. Combination according to claims 1 to 4, wherein
a. said first open reading frame additionally comprises,
i. one or more immunological tags for detection,
b. and said one or more nucleic acids additionally comprises (i) a selectable marker gene for expression in mammalian cells.

6. Combination according to claims 1 to 5, wherein
a. said promoter is selected from the group of
human cytomegalovirus (CMV) immediate-early promoter/enhancer, retroviral long terminal repeat (LTR) promoter/enhancer;
b. said cell surface targeting signal is selected from the group of
murine Ig κ-chain leader sequence, a mammalian immunoglobuline leader sequence, a leader sequence of retroviral envelope proteins, a leader sequence of other viral envelope proteins and a leader sequence of any mammalian type I transmembrane protein or secreted protein;
c. said transmembrane anchoring domain is selected from the group of
platelet-derived growth factor receptor transmembrane domain (PDGFR-TM), a transmembrane anchoring domain of retroviral envelope proteins, transmembrane anchoring domain of other viral envelope proteins, and of any mammalian type I transmembrane protein;
d. said group-specific antigen (gag) is selected from the group of
murine leukemia virus (MLV) gag, the group of γ-retroviral gag proteins, the group of the human immunodeficiency I virus (HIV-I), the group of gag proteins, and any gag protein of the family of *Retroviridae*;
e. said immunological tag is selected from the group of
hemagglutinin A epitope tag, myc epitope, or FLAG epitope.

7. Kit comprising a combination according to claims 1 to 6.

8. Method for producing a medicament or vaccine comprising the steps of
a. transferring the combination of the one or more nucleic acids according to claims 1 to 7 into a eukaryotic cell line,
b. incubating the cells,
c. harvesting the supernatant, and
d. isolating the Aβ-retroparticles from the supernatant.

9. Pharmaceutical composition comprising Aβ-retroparticles produced according to claim 8.

10. Pharmaceutical composition comprising,
Aβ-retroparticles comprising the following proteins or peptides,
(i) an amyloid peptide, (ii) cell surface targeting signal (iii) a transmembrane anchoring domain, and (iv) a group-specific antigen (gag).

11. Pharmaceutical composition according to claim 9 to 13, wherein said amyloid peptide is between 42 amino acids and 5 amino acids long.

12. Pharmaceutical composition according to claims 9 to 11, said amyloid peptide has an amino sequence selected from the group of SEQ ID NO. 4

13. Pharmaceutical composition according to claims 9 to 12, wherein the Aβ-retroparticles additionally comprise, one or more immunological tags for detection.

14. Pharmaceutical composition according to claims 9 to 13, wherein the composition is devoid of nucleic acids and/or free of a viral envelope protein.

15. Pharmaceutical composition according to claims 9 to 14, wherein the composition is for the treatment of a diseases associated with Aβ-aggregation.


SEQUENCE LISTING

<110>  Paul-Ehrlich-Institut Bundesamt für Sera und Impfstoffe

<120>  Methods and substances for the treatment of Alzheimer's

<130>  N1936 EP BLN

<160>  8

<170>  PatentIn version 3.4

<210>  1
<211>  120
<212>  PRT
<213>  Artificial

<220>
<223>  none

<400>  1

Met Glu Thr Asp Thr Leu Leu Leu Trp Val Leu Leu Leu Trp Val Pro
1               5                   10                  15

Gly Ser Thr Gly Asp Tyr Pro Tyr Asp Val Pro Asp Tyr Ala Gly Ala
            20                  25                  30

Gln Pro Ala Asp Ala Glu Phe Arg His Asp Ser Gly Tyr Glu Val His
        35                  40                  45

His Gln Ile Glu Gly Arg Pro Arg Leu Gln Val Asp Glu Gln Lys Leu
    50                  55                  60

Ile Ser Glu Glu Asp Leu Asn Ala Val Gly Gln Asp Thr Gln Glu Val
65                  70                  75                  80

Ile Val Val Pro His Ser Leu Pro Phe Lys Val Val Val Ile Ser Ala
                85                  90                  95

Ile Leu Ala Leu Val Val Leu Thr Ile Ile Ser Leu Ile Ile Leu Ile
            100                 105                 110

Met Leu Trp Gln Lys Lys Pro Arg
        115                 120

<210>  2
<211>  21
<212>  PRT
<213>  Mus musculus

<400>  2

Met Glu Thr Asp Thr Leu Leu Leu Trp Val Leu Leu Leu Trp Val Pro
1               5                   10                  15

Gly Ser Thr Gly Asp
            20

<210>  3
<211>  9
<212>  PRT
<213>  Influenza A virus

<400>  3

Tyr Pro Tyr Asp Val Pro Asp Tyr Ala
1               5

<210>  4
<211>  15
<212>  PRT
<213>  Homo sapiens

<400>  4

Asp Ala Glu Phe Arg His Asp Ser Gly Tyr Glu Val His His Gln
1               5                   10                  15

<210>  5
<211>  10
<212>  PRT
<213>  Homo sapiens

<400>  5

Glu Gln Lys Leu Ile Ser Glu Glu Asp Leu
1               5                   10

<210>  6
<211>  49
<212>  PRT
<213>  Homo sapiens

<400>  6

Ala Val Gly Gln Asp Thr Gln Glu Val Ile Val Val Pro His Ser Leu
1               5                   10                  15

Pro Phe Lys Val Val Val Ile Ser Ala Ile Leu Ala Leu Val Val Leu
            20                  25                  30

Thr Ile Ile Ser Leu Ile Ile Leu Ile Met Leu Trp Gln Lys Lys Pro
        35                  40                  45

Arg

<210>  7
<211>  31
<212>  DNA
<213>  Artificial

<220>
<223>  none

<400>  7
gatcggccca gccggccgat gcagaattcc g                                    31


<210>  8
<211>  41
<212>  DNA
<213>  Artificial

<220>
<223>  none

<400>  8
gactccgcgg ccggccctcg atcgctatga caacaccgcc c                         41